# EUROPÄISCHE PATENTSCHRIFT

(11) **EP 2 128 152 B1**
(45) Veröffentlichungstag und Bekanntmachung des Hinweises auf die Patenterteilung: **16.11.2011**
(21) Anmeldenummer: 09450080.8
(22) Anmeldetag: 15.04.2009
(51) Int. Cl.: C07D 319/12, C08G 63/08

(54) **Verfahren zur Reinigung zyklischer Diester der L-bzw. D-Milchsäure**
Method for cleaning cyclic diesters of L and D lactic acid
Procédé de nettoyage de diester cyclique de l'acide lactique L ou D

(30) Priorität: 16.04.2008 AT 5992008
(43) Veröffentlichungstag der Anmeldung: 02.12.2009
(73) Patentinhaber: JUNGBUNZLAUER AUSTRIA AG, 1010 Wien (AT)
(72) Erfinder: Rafler, Gerald, 14473 Potsdam (DE); Rafler, Jutta, 14473 Potsdam (DE); Windsperger, Andreas, 3034 Maria Anzbach (AT); Edlauer, Robert, 2136 Laa a.d. Thaya (AT); Gaß, Josef, 2265 Drösong (AT)
(74) Vertreter: Ellmeyer, Wolfgang

(56) Entgegenhaltungen:
- EP-A- 0 630 371
- EP-A- 0 657 447
- EP-A- 0 893 462

## Beschreibung

Die Erfindung betrifft ein Verfahren zur Reinigung von L,L- bzw. D,D-Dilactid aus meso-Dilactid enthaltenden Stereoisomerengemischen, durch dieses Verfahren hergestelltes L,L- bzw. D,D-Dilactid sowie die Verwendung von so hergestelltem L,L-bzw. D,D-Dilactid zur Herstellung von hochmolekularen Polymilchsäuren.

### STAND DER TECHNIK

Die Herstellung von hochmolekularen PLA-Kunststoffen für die thermoplastische Verformung zu Fäden, Folien, Schäumen oder Formkörpern durch Ringöffnungspolymerisation der zyklischen Diester der L- bzw. D-Milchsäure erfordert hochreine Monomere. Die Synthese dieser Monomere für die Polymilchsäuresynthese erfolgt in einem mehrstufigen Prozess mit biotechnologischen und chemischen Verfahrensstufen, der im Wesentlichen Folgendes umfasst:
- Hydrolyse stärkehaltiger Substrate zu Glucose;
- Fermentation von Glucose zu L- bzw. D-Milchsäure;
- Polykondensation zu einer niedermolekularen Poly-L- bzw. Poly-D-milchsäure;
- zyklisierende Depolymerisation zu L,L- bzw. D,D-Dilactid;
- Feinreinigung der Rohdilactide.

Dieser mehrstufige Prozess der Herstellung von vorzugsweise Poly-L-milchsäure wird in einer Vielzahl von Patenten von Cargill Inc., USA (vgl. beispielsweise US 6.277.951, US 6.005.067, US 5.357.035, US 6.291.597, CA 2.128.509), Dainippon Ink & Chem., Japan (vgl. beispielsweise US 5.844.066, US 5.616.657, US 5.605.981, US 5.403.897), Mitsui Toatsu Japan (vgl. beispielsweise US 5.194.473), Neste Oy, Finnland (WO 98/36008). Brussels Biotec (vgl. beispielsweise GB 2.407.572, WO 98102480, DE 69905016, US 6.489.508, US 2004/0014991) oder Shimadzu, Japan (vgl. beispielsweise US 5.770.682, US 5.866.677, JP 7206851), beschrieben.

In EP 0893462 A2 und JP 2004149419 werden konventionelle physikalische Trennverfahren, wie sie in unzähligen chemischen Prozessen in gleicher oder ähnlicher Weise angewandt werden, an die Trennaufgabe für Rohlactide adaptiert. In EP 0893462 A2 wird Schutz für einen Prozess zur Herstellung und Reinigung von L,L-Dilactid beansprucht, wie er in dieser Form von Cargill Corp., der Anmelderin von EP 0893462 A2, mit hoher Redundanz bereits mehrfach beschrieben wurde. Auch in anderen Dokumenten werden prinzipiell ähnliche Prozesse zur Herstellung des Monomers und des Polymers beschrieben (vgl. beispielsweise JP 7206891, US 5770982, US 5866682, WO 199836008, GB 2407572, DE 10020898), da Thermodynamik und Kinetik der Bildung aliphatischer Polyester aus α-Hydroxycarbonsäuren ähnlich sind. Auch werden in diesen Patentschriften in der Mehrzahl mehrstufige destillative Trennverfahren (ausgenommen GB 2407572) zur Feinreinigung des L,L-Dilactids eingesetzt, wobei die Unterschiede vor allem die apparative Ausgestaltung des Depolymerisationsprozesses und der Ringöffnungspolymerisation des zyklischen Monomers betreffen. Hinsichtlich der Rektifikation wird analog EP 0893462 A2 auf bewährte Destillationstechniken zurückgegriffen. Konkret beschreibt EP 0893462 A2 die Herstellung eines Polylactids aus einem Rohlactidgemisch durch Reinigen des Rohlactidgemischs durch Destillation, wodurch zwei Fraktionen, nämlich eine niedersiedende Fraktion und eine mittelhochsiedende Fraktion, die gereinigtes Lactid enthält, entstehen, wobei keine Lösungsextraktion oder Umkristallisation des Rohlactidgemischs durchgeführt wird, und Umsetzen des gereinigten Lactids zur Bildung eines Polylactids. Kombination des physikalischen Trennprozesses mit einem chemischen Prozess, wie der gezielt gesteuerten Hydrolyse eines unerwünschten Beiproduktes zur Verbesserung der Trenneffizienz, wird weder in EP 0893462 A2 noch in den aufgeführten analogen Verfahren beschrieben.

In JP 2004149419 wird ein Prozess zur Herstellung von L,L-Dilactid beschrieben, bei dem Entwässerung der L-Milchsäure und ihre Polykondensation zu einer oligomeren L-Milchsäure sowie die zyklisierende Depolymerisation dieser niedermolekularen Poly-L-milchsäure als azeotrope Destillation in einem organischen Lösungsmittel durchgeführt werden. Das Monomer wird aus dem überschüssigen Lösungsmittel mittels Kristallisation abgetrennt. Weiterhin wird in JP 2004149419 ausgeführt, dass beim Waschen des isolierten Rohlactids mit Wasser carboxylgruppenhaltige Verunreinigungen, vorzugsweise Lactoylmilchsäure und höhere lineare Oligomere, sowie meso-Dilactid infolge ihrer höheren Löslichkeit in Wasser von L,L-Dilactid abgetrennt werden können. Die Zugabe des Wassers erfolgt zum Roh-Dilactid. Das Waschen eines Kristallisats zur Abtrennung von Mutterlauge und anderen Verunreinigungen ist gängige Praxis bei derartigen Reinigungsoperationen an Feststoffen .

Unabhängig von technologischen und apparatetechnischen Konzepten in den aufgeführten Patentschriften ist die Gewinnung eines polymerisationsfähigen L,L-Dilactids zentraler Bestandteil der beschriebenen Verfahren. Chemische und chirale Reinheit der aus fermentativ gewonnener L- bzw. D-Milchsäure nach deren Polykondensation und zyklisierender Depolymerisation erhaltenen zyklischen Diester sind essenziell für die Herstellung einer hochmolekularen Polymilchsäure mit Verformungs- und Nutzungseigenschaften, wie sie von Verarbeitern und Nutzern hinsichtlich Rheologie und thermischer Stabilität der Polymerschmelze sowie mechanischer Festigkeit und Wärmeformbeständigkeit des polymeren Festkörpers gefordert werden. Chirale Verunreinigungen beeinflussen auf molekularer Ebene die Taktizität der Polyester und damit speziell die mechanischen und thermischen Festkörpereigenschaften. Chemische Verunreinigungen, vor allem in Form linearer Oligomere, wirken bei der Ringöffnungspolymerisation als Kettenregler und verhindern die Bildung ausreichend hoher Molmassen.

D- bzw. L-Lactideinheiten in Poly-L- bzw. in Poly-D-milchsäure, die durch optisch verunreinigte Milchsäure, Racemisierung bei der Polykondensation und/oder Zyklisierung bzw. unvollständige Abtrennung bei der Aufarbeitung der Dilactide eingebracht wurden, wirken trotz ihrer chemisch gleichen Struktur wie Comonomere und beeinflussen vor allem die Taktizität der Polymere. Dies führt dann zu den bereits angeführten Beeinflussungen der Morphologie des polymeren Festkörpers mit den beobachteten makroskopischen Änderungen im Schmelz- und Erweichungsverhalten sowie ausgewählten mechanischen Eigenschaften. Dabei sind die morphologischen Änderungen direkt eine Funktion der Konzentration der stereoisomeren Verunreinigungen, wie beispielsweise von D.W. Grijpma et al., Makromol. Chemie 195, 1649 (1994); G. Perego et al., J. Appl. Polymer Sci. 59, 37 (1996); G. Schmack et al., J. Appl. Polymer Sci. 73, 2785 (1999); oder R. E. Drumright et al., Adv. Mater. 12(23), 1841 (2000); und R. Auras et al., Macromol. Biosci. 4 835 (2004) gezeigt wird.

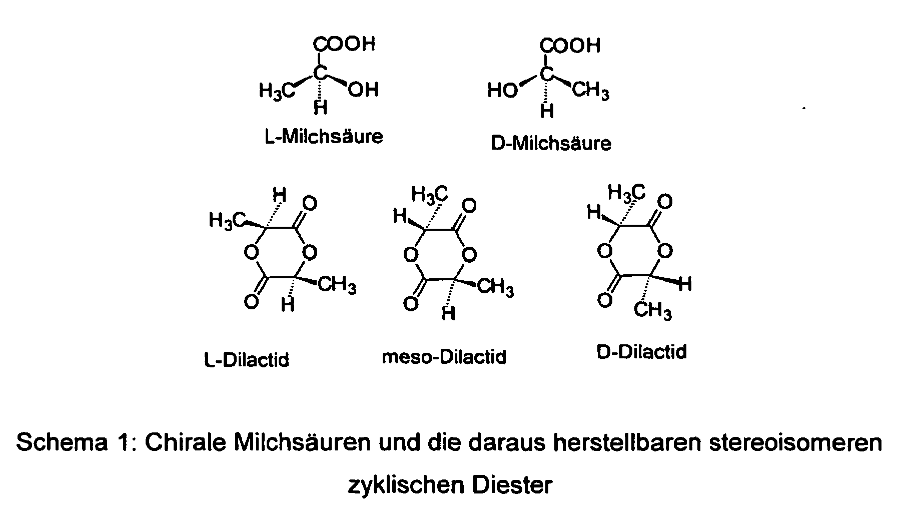

Die stereoisomeren Dilactide sind trotz gleicher chemischer Struktur als Verunreinigungen der strukturbestimmenden Hauptkomponente aufzufassen, und sie müssen vor der eigentlichen Polymersynthese möglichst vollständig abgetrennt werden. Selbst bei der gezielten Herstellung von strukturidentisch modifizierten Polymeren der Milchsäure (PLA oder PDLA (Poly-D-lactid)) durch Zusatz von D,D- oder meso-Dilactid bei PLA bzw. L,L- oder meso-Dilactid bei PDLA als Comonomere müssen die Ausgangsmonomere isomerenfrei sein, um Schwankungen in der Zusammensetzung und damit auch bei den Materialeigenschaften des Endprodukts zu vermeiden. Als Quellen für diese störenden Beimischungen des L,L- bzw. D,D-Dilactids kommen sowohl die betreffende L- bzw. D-Milchsäure als auch die chemischen Verfahrensschritte zur Herstellung der Dilactide in Frage. Ursprünglich in der Milchsäure vorhandene enantiomere Verunreinigungen der L- bzw. D-Milchsäure bleiben in dem durch Polykondensation hergestellten niedermolekularen Zwischenprodukt erhalten. Zusätzlich dazu können nach US 6.277.951 während der Polykondensation die optischen Antipoden durch Racemisierung nachgebildet werden. Angezeigt wird dies dann in der folgenden Verfahrensstufe durch den Anstieg des meso-Dilactidgehalts in dem durch zyklisierende Depolymerisation von niedermolekularer Poly-L-milchsäure gebildeten L,L-Dilactid. So wird in der US 6.277.951 ausgeführt, dass der meso-Dilactidanteil von ca. 5 auf 11 % ansteigt, wenn die Molmasse der zur Depolymerisation eingesetzten niedermolekularen Poly-L-milchsäure durch Verlängerung der Polykondensationszeit oder Erhöhung der Temperatur von 600 g/mol auf 3000 g/mol erhöht wird. Andererseits wird jedoch in den österreichischen Patentanmeldungen Nr. AM 1837/2007 und AM 1838/2007 gezeigt, dass höhere Molmassen der zur Depolymerisation eingesetzten Poly-L-milchsäure sich vorteilhaft auf Prozessgeschwindigkeit sowie Ausbeute und chemische Reinheit des L,L-Dilactids auswirken. So konnte durch Erhöhung der zahlenmittleren Molmasse der zur zyklisierenden Depolymerisation eingesetzten Poly-L-milchsäure von 800 g/mol auf 6100 g/mol die Depolymerisationsgeschwindigkeit um das 9fache gesteigert werden. Der Anteil linearer Oligomere, vor allem von Lactoylmilchsäure (lineares Dimer der Milchsäure), wurde ebenfalls durch Erhöhung der Molmasse der zur Depolymerisation eingesetzten Poly-L-milchsäure signifikant reduziert. In AM 1837/2007 und AM 1838/2007 wird dies anhand des [COOH]-Gehalts sowie des Drehwinkels der gebildeten Dilactide nachgewiesen, deren Werte für die jeweils ungereinigten Dilactide in Abhängigkeit von der Molmasse der zur Depolymerisation eingesetzten Ausgangs-PLA ermittelt wurden. Höhere Molmassen der eingesetzten Poly-L-milchsäure ergaben niedrigere Carboxylgruppenkonzentrationen sowie höhere optische Drehwinkel bei den abdestillierten Rohlactiden. Erreicht werden die höheren mittleren Molmassen der Polymilchsäuren in AM 1837/2007 und AM 1838/2007 durch den Einsatz von löslichen bzw. nanopartikulären Polykondensationskatalysatoren, die auch die zyklisierende Depolymerisation der Polymilchsäuren zu den Dilactiden beschleunigen und die im Gegensatz zu den in US 6.277.951 und US 5.844.066 beschriebenen Sn(II)-Verbindungen keine vermehrte meso-Dilactid-Bildung verursachen.

Der Anteil der stereoisomeren Dilactide in den Depolymerisationsbrüden wird von der Konzentration des optischen Antipoden im Polymer bestimmt, wenn Racemisierungen in der Depolymerisationsstufe ausgeschlossen werden können. Bei der zyklisierenden Depolymerisation von Poly-L-milchsäure mit D-Monomeranteilen im Makromolekül lässt sich bei vollständigem Umsatz und mit p für den L-Lactidgehalt die Verteilung der 3 Stereoisomere im Destillat nach Gleichung (1a) bis (1 c) berechnen:

L,L-Dilactid: p² (1a)

meso-Dilactid: 2p*(1-p) (1b)

D,D-Dilactid: (1-p)² (1c)

Während die Bildung von D,D-Dilactid bei D-Anteilen unter 10 % (entspricht p > 0,9 für den L-Monomeranteil) zu vernachlässigen ist, kann meso-Dilactid in diesem Bereich in Mengen gebildet werden, die die Ausbeute an L,L-Dilactid signifikant reduzieren und seine Feinreinigung erheblich erschweren.

Auch während des Depolymerisationsprozesses selbst können noch unerwünschte stereoisomere Verunreinigungen, vor allem meso-Dilactid, gebildet werden, die die Stoffbilanz und damit die Wirtschaftlichkeit des Prozesses zusätzlich belasten.

Stereoisomere sowie linearkettige Anteile müssen aus dem L,L-Dilactid abgetrennt werden, da sie die molekularen und makroskopischen Eigenschaften der polymeren L- bzw. D-Milchsäure in unerwünschter Weise beeinflussen. So führen D-Lactid-Monomereinheiten schon in geringen Konzentrationen zu einer starken Schmelzpunktdepression, und auch die mechanischen Eigenschaften weisen bei Poly-L-milchsäure eine hohe Abhängigkeit vom D-Lactidgehalt auf (vgl. Tabelle 1). Dabei ist es zunächst nicht entscheidend, ob die D-Isomer-Strukturen durch D,D- oder meso-Dilactid in das Polymer eingeführt werden. Beide stören die Taktizität des Polymers in gleicher Weise.

**Tabelle 1: Thermische und mechanische Eigenschaften von Poly-L-milchsäuren in Abhängigkeit vom D-Milchsäureanteil nach D. W. Grijpma, A. J. Pennings, Makromol. Chemie 195, 1649 (1994).**

| **D-Lactid [Mol-%]** | **Tₘ [°C]** | **Zugfestigkeit [MPa]** | **Schlagzähigkeit (kJ/m²]** |
|---|---|---|---|
| 0 | 192,1 | 59,5 | 12,7-13,5 |
| 0,3 | 184,2 | 63,0 | 15,0-15,8 |
| 0,5 | 183,3 | 66,7 | 36,7 |
| 1,0 | 178,9 | 65,2 | 26,3 |
| 5,0 | 150,7 | 56,9 | 8,9-9,3 |
| 9,1 | 133,1 | 59,3 | 10,3-12,4 |

Infolge der relativ geringen Nebenvalenz-Wechselwirkungskräfte bei aliphatischen Polyestern sind die Materialeigenschaften in einer für diesen Polymertyp typischen Weise in einem relativ weiten Bereich von der Molmasse abhängig. Dies bedeutet, dass für PLA-Materialien mit ausreichendem bis hohem Niveau der mechanischen Eigenschaften, wie es für Fasern, Folien oder Formkörper auf Basis von PLA gefordert ist, hochmolekulare Polyester einzusetzen sind, um die geforderten mechanischen Parameter von Festigkeit und Zähigkeit bei möglichst geringem Materialeinsatz zu erreichen. Auch für spezielle Verarbeitungstechniken, wie das Schäumen von Polymerschmelzen (Verarbeitung zu Trays), sind sehr hochmolekulare Polymere gefordert. Hier ist die für den Verarbeitungsprozess notwendige hohe Schmelzviskosität Ursache für die erforderlichen hohen Molmassen der Polymere.

Lineare Oligomere, insbesondere die dimere Lactoylmilchsäure, wirken infolge ihres Hydroxylgruppengehalts als so genannte Coinitiatoren, d.h. als Startzentren für das Kettenwachstum. Die Molmasse der gebildeten Polymere ist eine Funktion der Anzahl dieser Startzentren und damit direkt proportional zur OH-Gruppen-Konzentration. Bereits bei relativ geringen Konzentrationen können OH-Gruppen die Molmasse der PLA soweit erniedrigen, dass der Polyester nur noch begrenzt, z.B. für materialtechnisch anspruchlose Anwendungen, eingesetzt werden kann (vgl. die Tabellen 2 und 3).

**Tabelle 2: Molmasse in Abhängigkeit von der Konzentration OH-gruppenhaltiger Verunreinigungen (Daten aus US 5.844.066).**

| **T [°C]** | **OH-Gruppen [mÄqu./mol]** | **M [g/mol]** |
|---|---|---|
| 173 | 2, 52 | 77.500 |
| 173 | 4,45 | 40.100 |
| 186 | 2,38 | 72.100 |
| 186 | 3,90 | 37.800 |
| 199 | 2,48 | 74.900 |
| 199 | 3,98 | 39.400 |

**Tabelle 3: Molmasse in Abhängigkeit von der Konzentration OH-gruppenhaltiger Verunreinigungen (Daten aus WO 2005/056509).**

| **OH-Gruppen in Form von Milchsäure [µÄqu./g]** | **Mₙ [g/mol]** |
|---|---|
| 10 | 111.200 |
| 13 | 79.300 |
| 18 | 48.600 |
| 43 | 23.300 |
| 60 | 15.300 |
| 121 | 13.200 |

Auch die Hydrolysestabilität und damit ausgewählte spezifische Gebrauchs- sowie Abbaueigenschaften der aliphatischen Polyester auf Basis von Milchsäure sind eine Funktion der Festkörpereigenschaften der Polymere. Wie die signifikanten Unterschiede in der Abbaugeschwindigkeit der teilkristallinen Poly-L-lactide und Polyglykolide (PGA) zeigen (vgl. beispielsweise M. Szycher: "High Performance Biomaterials", Technomic Publishing Co. Inc., 1991; S. Vainionpää et al., Prog. Polymer Sci. 14, 679 (1989); D. W. Grijpma et al., Macromol. Chemie 195, 1633 (1994)), dominiert zwar der Einfluss der chemischen Struktur, aber auch bei geringeren Strukturunterschieden als denen zwischen PLA und PGA werden signifikante Differenzierungen im Abbauverhalten beobachtet. Unter In-vitro-Bedingungen werden resorbierbare Copolyester der L-Milchsäure, die durch Copolymerisation von L,L-Dilactid mit geeigneten, für die inkorporale Anwendung zertifizierten Comonomeren synthetisiert werden, deutlich schneller abgebaut als unmodifizierte PLA. Zu diesen Comonomeren für die Modifizierung von PLA gehören dabei auch die Stereoisomere des L,L-Dilactids, und auch Poly(L-co-D,L-lactide) unterschiedlichster Comonomerzusammensetzung werden rascher hydrolytisch abgebaut als unmodifiziertes PLA (vgl. beispielsweise L. E. Claes et al., Biomaterials 17, 1621 (1996); J. C. Middleton et al., Biomaterials 21, 2335 (2000); Firmendaten zu RESOMER-LR-Typen der Boehringer Ingelheim Pharma GmbH & Co, M. Pickard, Diss. TU Berlin 1996; D. W. Grijpma et al., Macromol. Chemie 195, 1633 (1994)).

Chirale und chemische Reinheit der Dilactide sind infolge ihrer extremen Beeinflussung molekularer Parameter (Molmasse und ihre Verteilung) und makroskopischer Eigenschaften der Polymere grundlegende Voraussetzungen für die Herstellung hochwertiger PLA- bzw. Poly-D-lactid- (PDLA-) Kunststoffe. Dementsprechend betreffen Herstellung und Reinigung der Monomere einschließlich der eingesetzten Apparatetechnik neben der eigentlichen Polymersynthese sowie der Stabilisierung der Polymere viele der beanspruchten Schutzrechte innerhalb des Gesamtprozesses.

Die Angaben zur Isomerenverteilung in den Rohlactiden aus der Depolymerisation schwanken naturgemäß in einem weiten Bereich, da die Verteilung sowohl von der optischen Reinheit der eingesetzten Milchsäuren als auch von technologischen Parametern bei Polykondensation und zyklisierender Depolymerisation abhängt. So wird in EP 893.462 ein meso-Dilactidgehalt von ca. 5 -11 % in Abhängigkeit von der zur Depolymerisation eingesetzten Poly-L-milchsäure angegeben. Besonders starke meso-Dilactidschwankungen in einem Bereich von 2-27 % werden in US 543086 beschrieben, wobei eine starke Aktivierung der meso-Isomerenbildung durch Alkalimetallsalze beobachtet wird. Dies ist besonders zu beachten, wenn während der Fermentation mit Alkalimetallhydroxiden neutralisiert und die Milchsäure nicht über einen Elektrodialyseprozess, sondern über Freisetzung mit Schwefelsäure und Fällung mit Kalkmilch gewonnen wird.

Für die Feinreinigung der Rohlactide werden zumeist destillative Reinigungsverfahren unter Vakuum beschrieben. Aber auch Fest/Flüssig-frennprozesse werden für die Dilactidreinigung angewandt. Bei der destillativen Feinreinigung ist zu berücksichtigen, dass die Siedepunkte der Dilactide mit Werten zwischen 256 und 260 °C angegeben werden (vgl. beispielsweise D. R. Witzke, Diss. Michigan State University, 1997; D. R. Witzke et al., Macomolecules 30, 7075 (1997)). Eine vollständige bzw. weitgehende Abtrennung des die thermischen Polymereigenschaften besonders negativ beeinflussenden meso-Dilactids vom L,L- bzw. D,D-Enantiomer ist auf destillativem Weg trotz geringer Dampfdruckunterschiede nicht möglich. Dies zeigen auch die Schmelzpunkte der wenigen marktverfügbaren PLA-Kunststoffe, die mit 152 bis 165 °C deutlich unter den für eine isomerenfreie PLA ermittelten Schmelzpunkten von 175 < Tₘ < 185 °C liegen (vgl. H. R. Kricheldorf, Chemosphere 43, 49 (2001)).

In der EP 630.371 wird eine zweistufige Destillation beschrieben, bei der das mit Milchsäure, linearen Oligomeren und Wasser sowie den Stereoisomeren belastete L,L-Dilactid einer ersten Kolonne zugeführt wird, wobei die Einspeisung der Dämpfe aus der Depolymerisationsstufe in einer mittleren Kolonnenstufe erfolgt. Die Kolonnentemperatur beträgt am Boden 180 °C, das Vakuum 40 mbar. Über den Kopf der Destillationskolonne werden die leichter siedenden Bestandteile (Wasser, Milchsäure) der Depolymerisationsbrüden abgeführt. Die Lactide werden in der Mitte der Kolonne unterhalb des Dampfeinlasses abgenommen und in eine zweite Kolonne zur nochmaligen Rektifikation bei 180 °C und 30 mbar übergeführt. Der Kolonnensumpf, der vor allem aus höhersiedenden linearen Oligomeren besteht, wird zur nochmaligen Zyklisierung zum Depolymerisationsreaktor rückgeführt.

In der WO 2005/056509 wird hinsichtlich der Zufuhr der Dilactiddämpfe zur Fraktionierkolonne eine ähnliche Verfahrensweise wie in EP 630.371 angewandt. Auch hier werden die Depolymerisationsdämpfe direkt der Fraktionierkolonne oberhalb des Kolonnenbodens zugeführt und zunächst eine niedrig siedende Fraktion aus Wasser, Milchsäure und Dilactid am Kolonnenkopf sowie eine höher siedende Fraktion aus Dilactid und linearen Oligomeren am Boden abgenommen. Aus den Dämpfen der Kopffraktion wird Dilactid durch fraktionierte Kondensation gewonnen und zur nochmaligen Fraktionierung in die Kolonne rückgeführt. Die höhersiedende Fraktion wird direkt wieder dem Depolymerisationsreaktor zugeführt. Für die Trennung geeignet sind Füllkörper- und Bodenkolonnen, wobei nach WO 2005/056509 Füllkörperkolonnen wegen der größeren Kontaktfläche für den Dampf/Flüssig-Austausch vorzuziehen sind. Zur Vermeidung von Folgereaktionen in der Kolonne werden Temperaturen im Bereich 150 bis 160 °C sowie Arbeiten unter einem reduzierten Druck von 20 bis 50 mbar empfohlen. Auch Inertgasbedingungen sind anzuwenden, um thermooxidative Belastungen zu vermeiden.

Nach dem offenbarten Rektifikationsverfahren werden Lactide mit einem Lactidgehalt von 99,0 bis 99,5 % bei einem OH-Gruppengehalt von 10 bis 20 µÄqu./g erhalten. Für die Herstellung einer hochmolekularen PLA soll jedoch der OH-Gruppengehalt 10 µÄqu./g nicht übersteigen. Zur Reduktion OH- und COOH-gruppenhaltiger Verunreinigungen können geeignete Fänger eingesetzt werden, die Reste der endgruppenhaltigen Verunreinigungen absorptiv oder chemisch binden und somit aus dem Lactid entfernen.

Auch in der EP 893.462 werden die verunreinigten Dilactiddämpfe aus der Depolymerisation in einer Kolonne in zwei Fraktionen aufgetrennt. Am Kolonnenkopf werden analog zu EP 630.371 und WO 2005/056509 Wasser und L-Milchsäure als Hauptkomponenten sowie Dilactid als geringer Fraktionsanteil abgenommen. Die mittlere Fraktion enthält das noch verunreinigte Lactidisomerengemisch als Hauptbestandteil. Die Hauptmenge des Lactidisomerengemischs, die vorwiegend aus L.L-Dilactid besteht, wird als mittlere Fraktion im Seitenstrang aus der Kolonnen abgeführt. Der Lactidanteil in dieser Fraktion beträgt 85 bis 95 %. Die Feinreinigung erfolgt ebenfalls destillativ, wobei wiederum zwei Fraktionen aus der Fraktionierkolonne abgetrennt werden.

Depolymerisationsreaktor und Fraktionierkolonne können getrennt betrieben werden. Zur Verbesserung der Wirtschaftlichkeit des Verfahrens (Erhöhung der Ausbeute, Verringerung des Investitionsaufwands) ist aber auch eine Integration von Reaktor und erster Kolonne möglich. Ein solches integriertes System von Reaktor und Kolonne wird beispielsweise in CA 2128509 und EP 893.462 beschrieben. Das Rohlactid aus der Depolymerisationsstufe kann direkt in dampfförmiger Form oder nach Kondensation als schmelzflüssige Phase der Fraktionierkolonne zugeführt werden (EP 722.469).

Neben der bevorzugten destillativen Aufreinigung der Rohlactide werden auch aufwändige Kristallisationsverfahren zur Feinreinigung angewandt. Sie sollen insbesondere auch eine Auftrennung des Lactidisomerengemischs und insbesondere die Abtrennung des meso-Dilactids ermöglichen. So wird in der GB 2.407.572 durch eine mehrstufige Schmelzkristallisation eine nahezu vollständige Abtrennung der meso-Isomere erreicht. Für stärker verunreinigtes Dilactid kann die Schmelzkristallisation mit einer Kristallisation aus Lösung bzw. mit einer Behandlung der Kristalle in einer wässrigen Phase kombiniert werden.

Wie in Schema 1 zu erkennen ist, unterscheiden sich die stereoisomeren Dilactide in der Anordnung der Methylgruppen relativ zur Ringebene des Cycloaliphaten. Diese konfigurativen Unterschiede zwischen L,L- bzw. D,D-Dilactid und dem meso-Dilactid beeinflussen auch die chemischen und physikalischen Eigenschaften der zyklischen Diester. Infolge der dominanten strukturellen Einflussfaktoren, die aus der Diesterstruktur resultieren, sind die konfigurativen Effekte der Methylgruppenanordnung zwar gering, aber feststellbar. Neben geringen Dampfdruckunterschieden zwischen L,L- bzw. D,D-Dilactid und den meso-Isomeren (vgl. D.R. Witzke, Diss. Michigan State University, 1997) werden auch Unterschiede in der Hydrolysestabilität und der Kristallisation beschrieben (vgl. GB 2.407.572). Diese geringere Hydrolysestabilität von meso- gegenüber L,L- bzw. D,D-Dilactid ist erwartungsgemäß bei den Polymeren infolge der Vervielfachung des Effekts durch die Polymerbildung extrem verstärkt. So ist Hydrolysegeschwindigkeit von Poly-D,L-lactid sehr viel höher als jene von PLA, wobei bei den Polymeren infolge des Charakters dieser Festkörperreaktion neben den strukturellen Faktoren auch morphologische Faktoren Ursache der beobachteten Unterschiede sind.

Die Hydrolyse der Lactide erfolgt zweistufig, wobei in einem ersten Schritt Lactoyl-milchsäure gebildet wird, die in einem zweiten Schritt durch Wasser zu Milchsäure gespalten wird (vgl. Schema 2). Beide Reaktionsschritte unterscheiden sich in ihrer Hydrolysegeschwindigkeit beträchtlich. Das Verhältnis zwischen der Geschwindigkeit der primären Ringhydrolyse zu Lactoylmilchsäure und jener der sekundären Spaltung zu Milchsäure beträgt ca. 100:1 (vgl. M. Pickard, Diss. TU Berlin 1996; M. M. Mhala et al., Indian J. Chem. 8, 243 (1970); C. Vidil et al., J. Chromatography A 711, 323 (1995)).

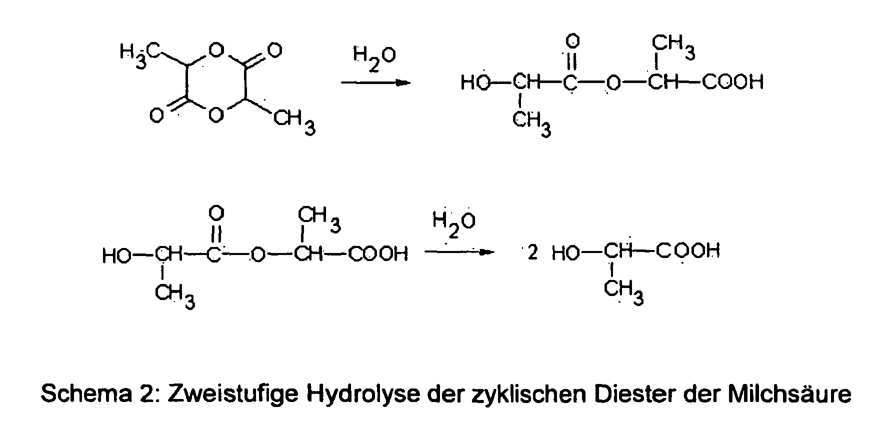

Infolge der unterschiedlichen sterischen Anordnung der Substituenten an den Ringsystemen und der damit verbundenen unterschiedlichen Zugänglichkeit der Estergruppen wird meso-Dilactid in der ersten Stufe der zweistufigen Dilactidhydrolyse rascher durch Wasser gespalten als das L- oder das D-Isomer.

In US 5.502.215 und EP 657.447 wird analog der Flüssigphasenkristallisation in GB 2.407.572 die höhere Hydrolysegeschwindigkeit des meso-Dilactids gegenüber L,L-Dilactid in Kombination mit seiner ebenfalls rascheren Auflösung in Wasser zur Abtrennung der meso-Isomere vom L,L-Dilactid genutzt. Dazu wird das Isomerengemisch in Abhängigkeit vom Isomerenverhältnis in fester oder geschmolzener Form mit Wasser versetzt und anschließend die L- bzw. D-Isomere als Feststoff von der wässrigen Phase mit dem meso-Dilactid bzw. seinen Hydrolyseprodukten abgetrennt.

### ZIEL DER ERFINDUNG

Aufgabe der Erfindung ist die Bereitstellung eines verbesserten Verfahrens zur Reinigung von L,L- bzw. D,D-Dilactid aus Stereoisomerengemischen unter Abtrennung von meso-Dilactid.

### OFFENBARUNG DER ERFINDUNG

Dieses Ziel wird durch ein Verfahren zur Reinigung von L,L- oder D,D-Dilactid aus einem Stereoisomerengemisch erreicht, wobei ein die Dilactid-Stereoisomere sowie höher und/oder niedriger siedende Bestandteile umfassendes Gemisch einem ersten Destillationsschritt unterzogen wird, um eine von den höher und niedriger siedenden Bestandteilen des Gemischs weitgehend gereinigte Fraktion der Dilactid-Stereoisomere zu erhalten, die anschließend einem zweiten Destillationsschritt unterzogen wird, um im Wesentlichen stereoisomerenfreies, gereinigtes L,L- oder D,D-Dilactid zu erhalten, das dadurch gekennzeichnet ist, dass vor dem zweiten Destillationsschritt Wasser zu der Fraktion der Dilactid-Stereoisomere zugesetzt wird, um meso-Dilactid selektiv zu Lactoylmilchsäure und/oder zu L- und D-Milchsäure zu hydrolysieren, von denen das L,L- oder D,D-Dilactid im zweiten Destillationsschritt in im Wesentlichen reiner Form abgetrennt wird.

Die Hydrolyse der Lactide erfolgt zweistufig, wobei in einem ersten Schritt, wie oben erwähnt und in Schema 2 dargestellt, Lactoylmilchsäure gebildet wird, die in einem zweiten Schritt durch Wasser zu Milchsäure gespalten wird. Beide Reaktionsschritte unterscheiden sich in ihrer Hydrolysegeschwindigkeit beträchtlich. Das Verhältnis der Geschwindigkeit von primärer Ringhydrolyse zu Lactoylmilchsäure zu sekundärer Spaltung in Milchsäure beträgt ca. 100:1. Infolge der unterschiedlichen sterischen Anordnung der Substituenten an den Ringsystemen und der damit verbundenen unterschiedlichen Zugänglichkeit der Estergruppen wird meso-Dilactid in der ersten Stufe der zweistufigen Dilactidhydrolyse rascher durch Wasser gespalten als das L,L- bzw. das D,D-Isomer. Dadurch wird eine Abtrennung von meso-Dilactid erreicht. Durch eine derartige gezielte Hydrolyse des rascher hydrolysierenden meso-Dilactids kann das L,L- oder das D,D-Isomer in weitaus - nämlich um etwa den Faktor 5 bis 10 - reinerer Form destillativ erhalten werden, als dies nach dem Stand der Technik möglich war, wie dies später anschaulich gezeigt wird.

Es kann auch vor dem zweiten Destillationsschritt eine aus dem Gehalt an meso-Dilactid im Isomerengemisch berechnete, stöchiometrische Menge oder ein geringer Überschuss an Wasser oder Wasserdampf zu der Fraktion der Dilactid-Stereoisomere zugesetzt werden, um meso-Dilactid selektiv zu Lactoylmilchsäure und/oder zu L- und D-Milchsäure zu hydrolysieren, von denen das L,L- oder D,D-Dilactid im zweiten Destillationsschritt in im Wesentlichen reiner Form abgetrennt wird.

Mit diesem Trennprozess wird erstmals ein physikalischer Trennprozess (Rektifikation) mit einem chemischen Prozess (Hydrolyse) bei der Dilactidreinigung kombiniert, wobei die geringere hydrolytische Stabilität des meso-Isomers gegenüber den chiralen Dilactiden gezielt und kontrolliert genutzt wird. Zeitpunkt und Mengenkontrolle des zugesetzten Wassers bzw. Wasserdampfes, wie sie hierin beansprucht werden, sind entscheidend, um unnötige Verluste an den Hauptkomponenten L,L-bzw. D,D-Dilactid durch unkontrollierte Hydrolyse zu vermeiden.

Dieser Reinigungsprozess für L,L- und D,D-Dilactide ist bestimmt von der Abhängigkeit der Zusammensetzung der Dilactide von der Qualität der eingesetzten Milchsäure (Enantiomerenzusammensetzung) sowie der Technologie der ersten chemischen Verfahrensstufe des Prozesses (Polykondensation der L- bzw. D-Milchsäure), die die Molmasse der zur zyklisierenden Depolymerisation eingesetzten niedermolekularen Poly-L- bzw. Poly-D-milchsäure bestimmt. Unkontrollierte Hydrolyse aller stereoisomeren Dilactide wird durch die Abtrennung von Milchsäure und Wasser als Kopfprodukt in der ersten Kolonne vermieden. Diese unkontrollierte Hydrolyse durch Wasser aus Nebenreaktionen der zyklisierenden Depolymerisation würde ansonsten zu signifikanten Ausbeuteverlusten führen und seine Wirtschaftlichkeit des Gesamtprozesses in Frage stellen. Bei der beanspruchten Verfahrensweise wird gezielt in Abhängigkeit vom meso-Dilactidgehalt Wasser bzw. Wasserdampf zudosiert, um vorzugsweise das sterisch bedingt besser zugängliche meso-Isomer (geringere Abschirmung der Estergruppen infolge Stellung der Methylgruppen zur Ringebene im Vergleich zu den chiralen Dilactiden, vgl. Schema 1) zu hydrolysieren und auf diese Weise als Sumpfprodukt (bei Partialhydrolyse zu Lactoylmilchsäure) am Boden bzw. als Kopfprodukt (bei Totalhydrolyse zu Milchsäure) der zweiten Kolonne abzunehmen.

Die Menge an Wasser sollte so gewählt werden, dass das meso-Dilactid vollständig aus dem Gemisch entfernt wird, ohne nennenswerte Megen an gewünschten L,L-oder D,D-Dilactid zu hydrolysieren. Die dazu benötigte Menge an Wasser kann anhand einer Analyse, z.B. mittels Gaschromatographie, des Gehalts an meso-Dilactid im Isomerengemisch berechnet werden, wobei vorzugsweise eine stöchiometrische Menge an Wasser - oder ein geringfügiger Überschuss (z.B. < 5%) - zugesetzt wird. Die Analyse des Dilactidgemischs erfolgt vorzugsweise nach der ersten Kolonne, um die Stereoisomerenzusammensetzung als Voraussetzung für die Wasserdosierung exakt nach der ersten Abtrennung des Rohlactids aus der Depolymerisation zu erfassen. Da die Wassermenge die Lage des Hydrolysegleichgewichts und damit auch die für die Hydrolyse erforderliche Reaktionszeit mitbestimmt, muss oftmals ein Kompromiss zwischen Reaktionsdauer und Ausbeute an gewünschten Ditactid-Isomer eingegangen werden. Erste und zweite bzw. dritte Trennkolonne unterscheiden sich signifikant in der Trenneffizienz. Die erste Kolonne dient bei Integration von Depolymerisationsreaktor und Kolonne aus wirtschaftlichen und prozesstechnischen Überlegungen vor allem der möglichst vollständigen Rückhaltung der höher siedenden Anteile und bei getrennter Apparateausführung der möglichst vollständigen Rückführung derselben in den Reaktor.

Das dem ersten Destillationsschritt unterzogene Gemisch kann aus einem Depolymerisationsreaktor zugeführt werden, oder die erste Kolonne kann mit einem solchen Reaktor fest verbunden oder darin integriert sein, was den apparativen Aufwand und den Raumbedarf verringern und die Verfahrensdauer senken kann. Die zur Depolymerisation eingesetzten Polymilchsäuren können dabei sowohl durch Polykondensation von L- bzw. D-Milchsäure als auch durch Polyumesterung von L- bzw. D-Milchsäureestern erhalten worden sein. Apparate-Integration erfolgt vorzugsweise bei der Depolymerisation von Polymilchsäuren mit zahlenmittleren Molmassen > 3.000 g/mol, da hier die Konzentration an linearen und zyklischen Verunreinigungen üblicherweise geringer ist als bei niedrigeren Molmassen.

In bevorzugten Ausführungsformen der Erfindung kann nach dem zweiten Destillationsschritt ein zusätzlicher, dritter Destillationsschritt zur weiteren Reinigung des L,L-oder D,D-Dilactids durchgeführt werden, um etwaige Spuren nicht abgetrennter höher (z.B. Milchsäure) oder niedriger (z.B. Lactoylmilchsäure) siedender Bestandteile des Gemischs vollständig zu entfernen.

Weiters werden die weiter zu reinigenden Fraktionen der Dilactid-Stereoisomere dem jeweils nächsten Destillationsschritt vorzugsweise in schmelzflüssiger Phase zugeführt, um einen intensiveren Kontakt mit dem zuzusetzenden Wasser als in der Gasphase zu ermöglichen. Das Wasser kann entweder flüssig oder als Wasserdampf zugesetzt werden, wobei Letzteres eine raschere Verteilung in der zu reinigenden Fraktion ermöglicht. Wird das Dilactidgemisch in kondensierter Phase mit Wasser bzw. Wasserdampf vermischt, so ist das kondensierte Gemisch anschließend vor der Durchführung des nächsten Destillationsschritts wieder zu verdampfen, beispielsweise mittels eines zwischengeschalteten Wärmetauschers.

Vorzugsweise wird das Wasser oder der Wasserdampf in eine Verbindungsleitung zwischen erster und zweiter Destillationsstufe zugesetzt, wobei die Dilactid-Stereoisomer-Fraktion und das Wasser oder der Wasserdampf in der Verbindungsleitung vorzugsweise mit einer Mischeinrichtung durchmischt werden, um intensiven Kontakt und ein einheitliches Gemisch zu ermöglichen, was für die Hydrolysereaktion förderlich ist.

Vorzugsweise werden ein oder mehrere oder auch alle Destillationsschritte in einer Rektifikationskolonne durchgeführt, um besonders gute Trennleistungen zu erzielen. Die Stelle des Abziehens der jeweiligen Fraktion des gewünschten Dilactids hängt von der Betriebsweise des erfindungsgemäßen Verfahrens (dis-, semi- oder kontinuierlich) und von der Art und Menge der im jeweiligen Destillationsschritt zu entfernenden Verunreinigungen ab, die wiederum (unter anderem) von den der Depolymerisation unterzogenen Milchsäurepolymeren abhängen. Ist das gewünschte Dilactid die am niedrigsten siedende Komponente, kann es auch bei kontinuierlichem oder semikontinuierlichem Betrieb am Kolonnenkopf abgezogen werden. Sind hingegen leichter flüchtige Bestandteile enthalten, wird an einer Stelle unterhalb des Kopfes abgezogen, wie dies später noch detaillierter ausgeführt wird.

Weiters werden die Destillationsschritte vorzugsweise bei einer Temperatur von 100 bis 200 °C, vorzugsweise 120 bis 180 °C, insbesondere 140 bis 160 °C, durchgeführt, um zu verhindern, dass das Dilactid (Fp.: 94-97 °C) im System kristallisiert oder unerwünschte Neben- oder Zersetzungsreaktionen auftreten. Diesen Temperaturen entsprechend werden in den Destillationsschritten Reaktionsdrücke von 1 bis 100 mbar, vorzugsweise 1 bis 50 mbar, insbesondere 3 bis 30 mbar, bevorzugt. Zudem können die Destillationsschritte in bevorzugten Ausführungsformen unter Inertgas durchgeführt werden, um thermooxidative Belastungen während der Destillation zu vermeiden.

Offenbart wird nach dem erfindungsgemäßen Verfahren erhaltenes, gereinigtes L,L-oder D,D-Dilactid, das vorzugsweise einen Schmelzpunkt von 94 bis 97 °C, einen Gehalt an meso-Dilactid von < 1,0 % und einen Carboxylgruppengehalt von < 10 µmol/g aufweist. Aus derartig reinem Dilactid-Stereoisomer können äußerst hochmolekulare Polymilchsäuren mit besonders vorteilhaften Eigenschaften, wie eingangs erwähnt, hergestellt werden.

Ebenfalls offenbart wird die Verwendung von so gereinigtem L,L- und/oder D,D-Dilactid zur Herstellung von Polymilchsäure mit einer zahlenmittleren Molmasse Mₙ von zumindest 75.000 g/mol und einer molekularen Uneinheitlichkeit M_{w}/Mₙ zwischen 1,6 und 3,0.

Zur Feinreinigung der Rohlactide durch reaktive Rektifikation in Abhängigkeit von ihrer chemischen und optischen Reinheit werden die im Folgenden beschriebenen technologischen und apparatetechnischen Verfahrensvarianten angewandt, wobei jede Variante zur Abtrennung des meso-Dilactids die kontrollierte Zudosierung von Wasser bzw. Wasserdampf nach der ersten Kolonne nutzt. Die Menge des zur Hydrolyse benötigten Wassers bzw. Wasserdampfes ergibt sich vorzugsweise aus dem gaschromatographisch bestimmten meso-Dilactid-Gehalt der Rohlactide. Zugeführt wird somit vorzugsweise eine Menge, wie sie entsprechend Schema 1 für den ersten Hydrolyseschritt zu Lactoylmilchsäure erforderlich ist. Die aus dem rascher hydrolysierbaren meso-Dilactid gebildete Lactoylmilchsäure wird in der zweiten Kolonne als höher siedende Fraktion zusammen mit den ursprünglich vorhandenen linearen Oligomeren in den Depolymerisationsdämpfen am Kolonnenboden abgenommen und wieder dem Depolymerisationsreaktor zugeführt oder partiell ausgeschleust.

Die der Reinigung im erfindungsgemäßen Verfahren unterzogenen Dilactide können, wie oben erwähnt, nicht nur durch Polykondensation von L- bzw. D-Milchsäure und deren Depolymerisation hergestellt werden, sondern es können auch entsprechend Gleichung (4) über Polyumesterung von Milchsäureestern hergestellte niedermolekulare Polyester zyklisierend depolymerisiert werden.

n CH₃CH(OH)COOR ↔ [-OCH(CH₃)CO-]ₙ + (n-1) ROH (4)

wobei R -C₂H₅, -C₃H₇ oder -C₂H₄OH ist.

Auch aus Milchsäure/Milchsäureester-Gemischen hergestellte Poly-L- oder Poly-D-milchsäure kann zur Dilactidsynthese und der erfindungsgemäßen Reinigung eingesetzt werden. Milchsäureester bilden eine Alternative zu den klassischen Verfahren der Milchsäurereinigung durch Membran- oder Fällungsprozesse. Sie können destillativ aus der Rohmilchsäure nach Veresterung mit den Alkanolen abgetrennt werden.

Als Depolymerisationsreaktoren werden Apparate mit hoher Wärme- und Stoffaustauschfläche eingesetzt, die die Polymilchsäuren aus der bei niedrigeren Temperaturen betriebenen Polykondensationsstufe möglichst rasch auf die übliche Depolymerisationstemperatur von 180 bis 240 °C erhitzen können und die eine rasche Abführung des gebildeten Dilactids ermöglichen. Die Geschwindigkeit der zyklisierenden Depolymerisationsreaktion ist trotz ihres Gleichgewichtscharakters infolge der Lage des Ring-Ketten-Gleichgewichts und des Arbeitens bei hoher Temperatur und reduziertem Druck hoch. Die pro Zeiteinheit abgeführte Rohlactidmenge ist von der Reaktantenkonzentration praktisch unabhängig, und für eine gegebene Molmasse der eingesetzten Polymilchsäure sowie einen gegebenen Katalysator ist sie damit nur eine Funktion des Stoffübergangs aus der flüssigen in die Gasphase sowie der Destillationsgeschwindigkeit der Rohlactiddämpfe in der ersten Kolonne. Eine ausreichend hohe Destillationsgeschwindigkeit, die die quantitative Abführung des gebildeten Dilactidgemischs ohne Behinderung erlaubt, ist nur bei kurzen mittleren Verweilzeiten und geringer Trennstufenzahl der ersten Kolonne möglich. Dies bedeutet, dass unter wirtschaftlichen und reaktionstechnischen Gründen die Trenneffizienz der ersten Kolonne auf Kosten einer hohen Destillationsgeschwindigkeit gegenüber der zweiten und dritten verringert ist.

Auch die Gestaltung der Depolymerisationsreaktoren als film- bzw. schleierbildende Horizontal- oder Vertikalreaktoren unterstützt die Abführung des gebildeten Rohlactids aus der PLA-Schmelze. Bei kleineren Kapazitäten können auch Rührreaktoren mit radialer oder vertikaler Durchmischung eingesetzt werden. Zur Unterstützung der Stoffübertragung in die Gasphase wird die Reaktion unter reduziertem Druck von 3 bis 30 mbar sowie vorzugsweise unter Inertgas durchgeführt.

Wird das im erfindungsgemäßen Verfahren zu reinigende Gemisch durch Depolymerisation von Polymilchsäuren mit zahlenmittleren Molmassen Mₙ < 3.000 g/mol und hohem Gehalt an unerwünschten Stereoisomeren erhalten, sind drei Destillationsschritte erforderlich.

Die apparative Auslegung der destillativen Reinigungsstufe, vor allem der Anzahl der Trennkolonnen sowie der Stelle von Brüdenein- und -ausgang, wird unter anderem von der Molmasse und der optischen Reinheit der zur Depolymerisation eingesetzten Poly-L- bzw. Poly-D-milchsäure bestimmt. Da bei Einsatz von höhermolekularen Polymilchsäuren der Anteil an aus den Depolymerisationsbrüden abzutrennenden Nebenprodukten entsprechend AM 1837/2007 und AM 1838/2007 geringer ist, reduziert sich auch der technologische und apparatetechnische Aufwand für die Feinreinigung der Rohlactide durch reaktive Fraktionierung. Die Ursache dieser Abhängigkeit der anzuwendenden Reinigungstechnologie von der Molmasse der eingesetzten Polymilchsäure ist auf Parallel- und Folgereaktionen der zyklisierenden Depolymerisation zurückzuführen. Erfolgt die Depolymerisation unter Einsatz eines sehr niedermolekularen Milchsäurepolymers, ist ein hoher Anteil von die spätere Polymerisation störenden Verunreinigungen im Rohlactid nachweisbar, die abgetrennt werden müssen. Verursacht werden diese Verunreinigungen durch Wasser, das infolge der geringen Molmassen und damit hohen Endgruppenkonzentrationen durch parallel ablaufende Polykondensationsprozesse noch in so hohen Anteilen bei der Depolymerisation gebildet wird, dass bereits gebildetes Dilactid in der Schmelze bzw. in den Lactiddämpfen oder Destillaten unkontrolliert zurächst zu Lactoylmilchsäure und dann weiter zu Milchsäure hydrolysiert wird.

Entsprechend Gleichung (2) werden aus n mol Milchsäure bei der Polykondensation (n-1) mol Wasser gebildet. Unter Berücksichtigung des Zusammenhangs von zahlenmittlerem Polymerisationsgrad und Reaktionsfortschrittsgrad in den Gleichungen (3a) und (3b) bedeutet dies beispielsweise, dass aus 900 g (10 mol) einer wasserfreien Milchsäure insgesamt 162 g Wasser gebildet werden, entsprechend 81 g bei Dimerisation oder ca. 146 g, wenn das lineare Dekamer mit Mₙ = 738 g/mol hergestellt wird. Für die gleiche Milchsäuremenge bei Herstellung eines linearen Oligomers mit Mₙ = 3.000 g/mol (entsprechend Pₙ = 41,6) müssen ca. 158 g Wasser aus dem System entfernt werden. 12 g Wasser stehen damit selbst beim Übergang von Pₙ = 10 auf Pₙ = 41,6 noch für die unkontrollierte Dilactidhydrolyse zur Verfügung. Das entspricht einem Hydrolysepotenzial von 0,67 mol/mol bei Abtrennung und Aufarbeitung des Dilactids.

n CH₃CH(OH)COOH ↔ [-OCH(CH₃)CO-]ₙ + (n-1) H₂O (2)

Pₙ=1/(1-p) (3a)

p = 1 - 1/Pₙ (3b)

Erkennbar ist der Einfluss der Molmasse der zur Depolymerisation eingesetzten niedermolekularen Poly-L- bzw. Poly-D-milchsäure an den COOH-Gruppen-Konzentrationen und den Drehwinkeln für polarisiertes Licht der gebildeten Dilactide, wie sie aus dem Depolymerisationsreaktor abgeführt werden. Dabei zeigen COOH-Gruppen und Drehwinkel unterschiedliche Verunreinigungen der rohen Dilactide an. Während die COOH-Gruppenkonzentration chemische Verunreinigungen, wie Milchsäure und lineare Oligomere, vorzugsweise Lactoyl-milchsäure, anzeigt, ist die optische Aktivität, gemessen am Drehwinkel, ein Maß für den Anteil chemischer und optischer Verunreinigungen des Dilactids. Über die optischen Drehwerte werden somit sowohl die Konzentration linearer Komponenten als auch die der Stereoisomere des L,L- bzw. D,D-Dilactids erfasst. Für die weitere Aufarbeitung der Dilactide sind somit bei Polykondensation und zyklisierender Depolymerisation solche Bedingungen anzustreben, bei denen die Carboxylgruppenkonzentrationen möglichst niedrig ([COOH] < 0,2 mmol/g) und der Betrag der optischen Drehwerte möglichst hoch ([α] > |-250°|) sind; wie in der folgenden Tabelle 4 veranschaulicht wird.

**Tabelle 4: Carboxylgruppengehalte und optische Drehwerte ungereinigter L,L-Dilactide in Abhängigkeit von der Molmasse der eingesetzten Poly-L-milchsäure.**

| **Mₙ [g/mol]** | **[COOH] [mmol/g)** | **[α] [°]** |
|---|---|---|
| 550 | 1,20 | -235 |
| 800 | 0,75 | -247 |
| 1600 | 0,70 | -250 |
| 2300 | 0,50 | -250 |
| 3500 | 0,21 | -255 |
| 4800 | 0,13 | -257 |
| 7500 | 0,08 | -260 |

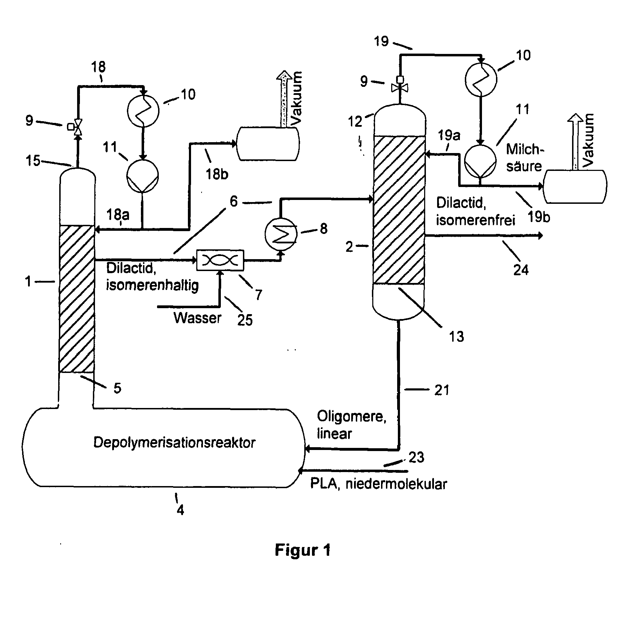

Die nach einer der drei im Folgenden beschriebenen und in den Fig. 1-3 schematisch dargestellten Verfahrensvarianten aufgearbeiteten, optisch aktiven Dilactide weisen Schmelzpunkte Tₘ von 96-97 °C. Carboxylgruppenkonzentrationen [COOH] von 5-10 µmol/g sowie Drehwerte [α] von -272° bis -282° für L,L-Dilactid bzw. 272° bis 281° für das D-Enantiomer auf. Die meso-Dilactid-Anteile in den erfindungsgemäß aufgearbeiteten Rohlactiden wurden mit 0,2 % ermittelt, wobei der meso-Stereoisomer-Gehalt in den Rohlactiden in Abhängigkeit von der Milchsäurequalität und der Polykondensations- und Zyklisierungstechnologie bei 3 bis 12 % lag. Zum Vergleich zeigt Tabelle 5 die Zusammensetzung ungereinigter L,L-Dilactide.

**Tabelle 5: Zusammensetzung ungereinigter L,L-Dilactide.**

| **L,L-Dilactid (unger.)** | **Tₘ [°C]** | **[COOH] [mmol/g]** | **[α] [°]** | **meso-Dilactid [%]** |
|---|---|---|---|---|
| Versuch A | 89-92 | 0,90 | -239 | 5,2 |
| Versuch B | 91-93 | 0,45 | -250 | 5,0 |
| Versuch C | 92-93 | 0,13 | -255 | 5,3 |
| Versuch D | 86-88 | 1,15 | -220 | 9,4 |

Die Destillation der Dilactide erfolgt, wie erwähnt, besonders bevorzugt bei Temperaturen von 140-160 °C und im Vakuum bei Drücken von 3-30 mbar. Als Destillationskolonnen werden deshalb vorzugsweise Rektifikations-, für die zweite und dritte Destillationsstufe vor allem Füllkörperkolonnen eingesetzt, die bei Arbeiten unter reduziertem Druck einen vergleichsweise geringen Druckabfall aufweisen. Darüber hinaus sind sie durch hohe Trenneffizienz und geringe Störanfälligkeit charakterisiert. Letzteres ist für den Trennprozess von besonders hoher Relevanz, da ein bei Temperaturen von ca. 50 °C leicht kristallisierendes Produkt destilliert wird.

Mit erfindungsgemäß gereinigtem Dilactid wurden Polymerisationsversuche unter diskontinuierlicher und kontinuierlicher Verfahrensweise durchgeführt, um seine Eignung für die Polymerherstellung sowie die Qualitätsparameter der Polymere zu prüfen. Für die diskontinuierlichen Polymerisationsversuche wurde eine Glasapparatur mit geschraubtem Blattrührer eingesetzt. Für die kontinuierlichen Polymerisationsversuche wurde ein zweistufiges apparatives System, bestehend aus einem Rührreaktor und einem Doppelschneckenextruder mit einem speziell auf diese Massepolymerisation abgestimmten Schneckendesign mit einem hohen Anteil an Knetelementen verwendet. In Gegenwart von Zinn(II)-octanoat konnten dabei in Abhängigkeit von den Polymerisationsbedingungen (Temperatur, Cokatalysatorzusatz, Stabilisierung) und der Verfahrensweise (diskontinuierlich im Rührgefäß, kontinuierlich im Doppelschneckenextruder) Polymere mit zahlenmittleren Molmassen im Bereich 40.000 g/mol < Mₙ < 175.000 g/mol bei einer Polydispersität von 1,5 < M_{w}/Mₙ < 3,0 synthetisiert werden. Die Schmelzpunkte der PLA-Muster lagen bei Tₘ = 170-183 °C und die Drehwinkel für polarisiertes Licht bei α = -157° bis -159°.

### KURZBESCHREIBUNG DER ZEICHNUNGEN

Die Fig. 1 bis 3 zeigen schematische Fließbilder von drei Dilactid-Reinigungsverfahren als Ausführungsformen der Erfindung. In den Figuren bedeuten die Bezugszeichen Folgendes:
- 1: Kolonne 1
- 2: Kolonne 2
- 3: Kolonne 3
- 4: Depolymerisationsreaktor
- 5: Kolonnenboden in Kolonne 1
- 6: Zuleitung von Kolonne 1 zu Kolonne 2
- 7: Mischer in Zuleitung 6
- 8: Heizung
- 9: Sperrventil
- 10: Wärmetauscher
- 11: Pumpe
- 12: Kolonnenkopf in Kolonne 2
- 13: Kolonnenboden in Kolonne 2
- 14: Seitenstrang von Kolonne 2 zu Kolonne 3
- 15: Kolonnenkopf in Kolonne 1
- 16: Kolonnenkopf in Kolonne 3
- 17: Kolonnenboden in Kolonne 3
- 18, 18a, 18b, 19, 19a, 19b, 20, 20a, 20b, 21, 21 a, 21 b, 22, 23, 24: Leitung

### BESCHREIBUNG BEVORZUGTER AUSFÜHRUNGSFORMEN

### Reaktive Fraktionierung von aus höhermolekularer Poly-L- bzw. Poly-D-milchsäure mit geringem Stereoisomerengehalt und einem Mₙ > 3000 g/mol erhaltenen rohen Dilactiden (vgl. Fig. 1)

Die Dämpfe der zyklisierenden Depolymerisation von höhermolekularer und mesoisomerenarmer Poly-L- bzw. Poly-D-milchsäure aus einem Depolymerisationsreaktor 4 werden einer direkt mit dem Depolymerisationsreaktor 4 verbundenen Kolonne 1 über den Kolonnenboden 5 zugeführt. Die niedrig siedenden Anteile der Depolymerisationsbrüden, die vor allem aus dem Dilactidgemisch und einem sehr geringen Anteil an L- bzw. D-Milchsäure und Wasser bestehen, werden als Kopfprodukt abgenommen, partiell kondensiert, und dem schmelzflüssigen Kopfprodukt wird zur Abtrennung von meso-Dilactid erhitztes Wasser oder Wasserdampf zudosiert. Die Zufuhr dieses isomerenhältigen Dilactids von Kolonne 1 zu Kolonne 2 erfolgt über die Zuleitung 6. Dieses Gemisch wird nach erneutem Verdampfen mithilfe einer Heizung 8, die sich in der Zuleitung 6 befindet, einer Kolonne 2 im mittleren Bereich zugeführt. Das Zuführen von Wasser oder Wasserdampf kann über eine Leitung 25 in einem Mischer 7, der in Zuleitung 6 vorhanden sein kann, erfolgen. Die Intensivierung des Kontakts zwischen dem Dilactidgemisch und dem zugeführten Wasser oder Wasserdampf erfolgt durch in die Zuleitung 6 eingebaute statische Mischer 7. Das Zumischen von Wasser oder Wasserdampf erfolgt somit zwischen Kolonne 1 und Kolonne 2 in einer Zuleitung 6, die Kolonne 1 und Kolonne 2 verbindet. In Kolonne 2 werden über den Kolonnenkopf 12 ein Milchsäure/Wasser-Gemisch und über den Kolonnenboden 13 lineare Oligomere, vor allem aus dem meso-Dilactid gebildete Lactoylmilchsäure, abgeführt. Das meso-isomerenfreie L,L- bzw. D,D-Dilactid wird als mittlere Fraktion aus Kolonne 2 abgenommen und in schmelzflüssiger Form direkt der Ringöffnungspolymerisation zugeführt bzw. kristallisiert und isoliert. Die höher siedende Fraktion aus Kolonne 2, die aus linearen Oligomeren der Milchsäure und einem geringen Anteil an Dilactid besteht, wird wieder dem Depolymerisationsreaktor 4 zugeführt, um sie erneut zyklisierender Depolymerisation zu unterziehen.

**Tabelle 6: Analysendaten für gereinigtes L,L-Dilactid (bei Rektifikation entsprechend Fig. 1).**

| **L,L-Dilactid (gereinigt)** | **Tₘ [°C]** | **[COOH] [µmol/g]** | **[α] [°]** | **meso-Dilactid [%]** |
|---|---|---|---|---|
| aus Versuch B | 95 | 8 | -272 | 0,25 |
| aus Versuch C | 96 | 6 | -274 | 0,20 |

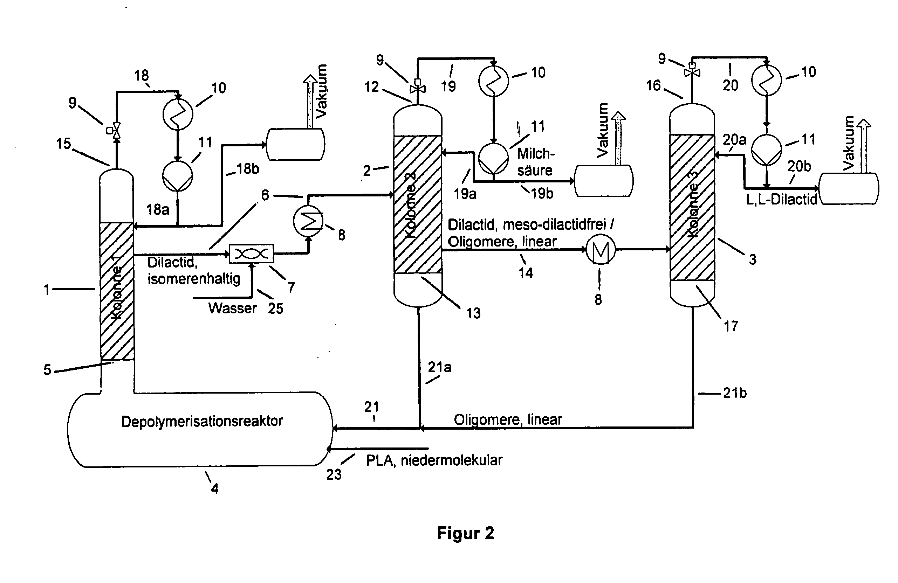

### Reaktive Fraktionierung von aus höhermolekularer Poly-L- bzw. Poly-D-milchsäure mit höherem Stereoisomerengehalt und einem Mₙ > 3000 g/mol erhaltenen rohen Dilactiden (vgl. Fig. 2)

Wird L,L- bzw. D,D-Dilactid mit höheren meso-Anteilen, die aus Poly-L- bzw. Poly-D-milchsäuren geringerer optischer Reinheit hergestellt wurden, nach dem erfindungsgemäßen Verfahren aufgearbeitet, so wird vorzugsweise eine zusätzliche Trennkolonne 3 (Kolonne 3 in Fig. 2) zur Rektifikation eingesetzt, um die Monomere auf die für die Herstellung eines hochwertigen Polylactids erforderliche chemische und optische Reinheit zu bringen. Entsprechend Fig. 2 werden die Dämpfe der zyklisierenden Depolymerisation einer direkt mit einem Depolymerisationsreaktor 4 verbundenen Kolonne 1 über den Kolonnenboden 5 zugeführt. Die niedriger siedenden Anteile der Depolymerisationsbrüden, die vor allem aus dem Dilactidgemisch und einem geringen Anteil an L- bzw. D-Milchsäure und Wasser bestehen, werden wie zuvor als Kopfprodukt abgenommen, partiell kondensiert und nach Zudosierung von erhitztem Wasser oder Wasserdampf zur schmelzflüssigen Phase in Zuleitung 6, die Kolonne 1 und Kolonne 2 verbindet, und erneutem Verdampfen mithilfe einer Heizung 8, die in der Zuleitung 6 vorgesehen ist, einer Kolonne 2 im mittleren Bereich zugeführt. Das Zuführen von Wasser oder Wasserdampf kann über eine Leitung 25 in einem Mischer 7, der in der Zuleitung 6 vorgesehen sein kann, erfolgen. Die Intensivierung des Kontakts zwischen dem Dilactidgemisch und dem zugeführten Wasser erfolgt durch in die Zuleitung 6 eingebaute statische Mischer 7. Das Zumischen von Wasser oder Wasserdampf erfolgt somit zwischen Kolonne 1 und Kolonne 2 in einer Zuleitung 6, die Kolonne 1 und Kolonne 2 verbindet. In Kolonne 2 werden über den Kolonnenkopf 12 ein Milchsäure/Wasser-Gemisch und den Kolonnenboden 13 lineare Oligomere, vorzugsweise aus dem meso-Dilactid gebildete Lactoylmilchsäure, über Leitungen 19 bzw. 21a abgeführt. Das weitgehend meso-isomerenfreie L,L- bzw. D,D-Dilactid wird als mittlere Fraktion (in Fig. 2 nicht maßstabsgetreu dargestellt) im Seitenstrang 14 aus Kolonne 2 abgenommen und einer Kolonne 3 zur Feinrektifikation zugeführt. Aus Kolonne 3 wird im Wesentlichen reines L,L- bzw. D,D-Dilactid als Kopfprodukt über Leitung 20 abgenommen und in schmelzflüssiger Form direkt der Ringöffnungspolymerisation zugeführt bzw. kristallisiert und isoliert. Die höher siedenden Fraktionen aus den Kolonnen 2 und 3, die aus linearen Milchsäure-Oligomeren und einem geringen Anteil an Dilactid bestehen, werden über Leitungen 21a, 21b, 21 wieder dem Depolymerisationsreaktor 4 zugeführt, um sie erneut zyklisierender Depolymerisation zu unterziehen.

**Tabelle 7: Analysendaten für gereinigtes L,L-Dilactid (bei Rektifikation entsprechend Fig. 2).**

| **L,L-Dilactid (gereinigt)** | **Tₘ [°C]** | **[COOH] [µmol/g]** | **[α] [°]** | **meso-Dilactid [%]** |
|---|---|---|---|---|
| aus Versuch B | 96 | 6 | -279 | 0,18 |
| aus Versuch C | 97 | 5 | -279 | 0,15 |

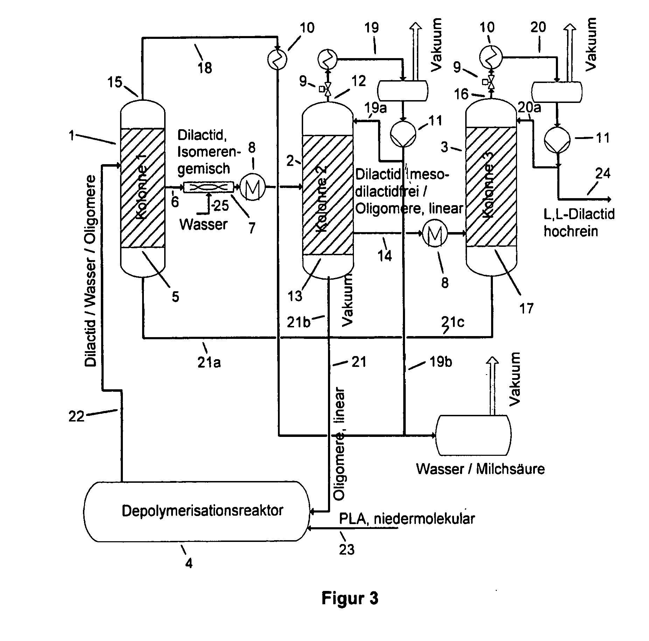

### Reaktive Fraktionierung von aus Poly-L- bzw. Poly-D-milchsäure mit einem Mₙ < 3000 g/mol und beliebiger optischer Reinheit erhaltenen rohen Dilactiden (vgl. Fig. 3)

Der in Fig. 3 dargestellte Rektifikationprozess ist zur Feinreinigung von Rohlactiden beliebiger Reinheit anzuwenden. Vorzugsweise ist er einzusetzen bei Dilactiden, die aus zyklisierender Depolymerisation von Polymilchsäure niedrigerer Molmasse und geringerer optischer Reinheit erhalten werden. Bei diesem Prozess für durch lineare Oligomere und Stereoisomere stärker verunreinigte Dilactide sind im Gegensatz zu den in Fig. 1 und 2 dargestellten Rektifikationsprozessen Depolymerisationsreaktor 4 und Rektifikationskolonne 1 getrennte apparative Einheiten, die durch eine Leitung 22 verbunden sind. Die Dilactiddämpfe aus einem Depolymerisationsreaktor 4 werden über eine Leitung 22 einer Kolonne 1 im mittleren Bereich zugeführt. Die niedriger siedenden Anteile der Depolymerisationsbrüden, die vor allem aus L- bzw. D-Milchsäure und Wasser bestehen, werden als Kopfprodukt abgenommen. Die mittlere Fraktion, die aus dem Dilactidgemisch und geringen Anteilen an linearen Oligomeren besteht, wird als Seitenstrang 6, der Kolonne 1 und Kolonne 2 verbindet, aus Kolonne 1 abgetrennt, partiell kondensiert und nach Zudosierung von erhitztem Wasser oder Wasserdampf, beispielsweise über eine Leitung 25 in einem Mischer 7, der in der Zuleitung 6 vorgesehen sein kann, zur schmelzflüssigen Phase und erneutem Verdampfen, beispielsweise mithilfe einer Heizung 8, die in der Zuleitung 6 vorgesehen sein kann, einer Kolonne 2 im mittleren Bereich zugeführt. Die Intensivierung des Kontakts zwischen dem Dilactidgemisch und dem zugeführten Wasser bzw. Wasserdampf erfolgt durch in die Zuleitung 6 eingebaute statische Mischer 7. Das Zumischen von Wasser oder Wasserdampf erfolgt somit zwischen Kolonne 1 und Kolonne 2 in einer Zuleitung 6, die Kolonne 1 und Kolonne 2 verbindet. In Kolonne 2 werden über den Kolonnenkopf 12 ein Milchsäure/WasserGemisch und über den Kolonnenboden 13 lineare Oligomere, vorzugsweise aus dem meso-Dilactid gebildete Lactoylmilchsäure, über Leitungen 19 bzw. 21b abgeführt. Das weitgehend meso-isomerenfreie L,L- bzw. D,D-Dilactid wird als mittlere Fraktion (in Fig. 3 nicht maßstabsgetreu dargestellt) im Seitenstrang 14 aus Kolonne 2 abgenommen und einer Kolonne 3 zur Feinrektifikation zugeführt. Der Austrag der mittleren Fraktionen aus den Kolonnen 2 und 3 erfolgt jeweils unterhalb der Zufuhr der Rohlactid- bzw. vorgereinigten Dilactiddämpfe in die jeweilige Rektifikationskolonne. Aus Kolonne 3 wird hochreines L,L- bzw. D,D-Dilactid als Kopfprodukt über Leitung 20 abgenommen und in schmelzflüssiger Form direkt einer Ringöffnungspolymerisation zugeführt bzw. kristallisiert und isoliert. Die höher siedenden Fraktionen aller drei Rektifikationskolonnen, die aus linearen Milchsäure-Oligomeren und einem geringen Anteil an Dilactid bestehen, werden über Leitungen 21a, 21b, 21c, 21 wieder dem Depolymerisationsreaktor 4 zugeführt, um sie erneut zyklisierender Depolymerisation zu unterziehen.

**Tabelle 8: Analysendaten für gereinigtes L,L-Dilactid (bei Rektifikation entsprechend Fig. 3).**

| **L,L-Dilactid, gereinigt** | **Tₘ [°C]** | **[COOH] [µmol/g]** | **[α] [°]** | **meso-Dilactid [%]** |
|---|---|---|---|---|
| Versuch E | 95 | 10 | -274 | 0,30 |
| Versuch F | 96 | 6 | -279 | 0,20 |
| Versuch G | 97 | 5 | -281 | 0,15 |
| Versuch H | 95 | 11 | -272 | 0,32 |

### BEISPIELE

Die Erfindung wird nun anhand der folgenden, nicht einschränkenden Beispiele detailliert beschrieben.

### Synthesebeispiel 1: Polykondensation von 85%iger L-Milchsäure

In einem flüssig-beheizten 25-1-Laborrührreaktor, ausgerüstet mit einem Ankerrührer, einer Mantelheizung, einem Bodenventil und einer temperierbaren Kolonne sowie mit Erfassung von Innen- und Manteltemperatur, wurden 15 kg einer 85%igen L-Milchsäure bei 120 bis 160 °C unter reduziertem Druck (brüdengesteuertes Temperatur-und Druckprogramm) entwässert und anschließen in Gegenwart einer Katalysatorkombination aus 2,0 x 10⁻⁴ mol/mol Dihydroxybis(ammoniumlactato)titan und 2 x 10⁻⁴ mol/mol SnCl₂ 4 h lang bei 190 °C polykondensiert.

| | |
|---|---|
| Ausbeute: | 10,2 kg |
| Mₙ: | 4800 g/mol |
| [COOH]: | 0,2 mmol/g |

### Synthesebeispiel 2: Polykondensation von wasserfreier L-Milchsäure

Analog Synthesebeispiel 1 wurden in einem flüssig-beheizten 25-1-Laborrührreaktor, ausgerüstet mit einem Ankerrührer, einer Mantelheizung, einem Bodenventil und einer temperierbaren Kolonne sowie mit Erfassung von Innen- und Manteltemperatur, 15 kg einer wasserfreien L-Milchsäure bei 160 °C unter reduziertem Druck in Gegenwart von 3 x 10⁻⁴ mol/mol Sn(oct)₂ polykondensiert.

| | |
|---|---|
| Ausbeute: | 11,5 kg |
| Mₙ: | 3500 g/mol |
| [COOH]: | 0,4 mmol/g |

### Svnthesebeispiel 3: Polyumesterung von Ethyl-L-lactat

1180 g Ethyl-L-lactat wurden in einer Glasapparatur mit Rührer, Außenheizung und temperierbarer Vigreuxkolonne bei 200 °C in Gegenwart von 3 x 10⁻⁴ mol/mol Di-hydroxybis(ammoniumlactato)titan polykondensiert. Das abgespaltene Ethanol wurde über die Kolonne abgeführt und kann für erneute Veresterungen von L-Milchsäure direkt verwendet werden. Zur vollständigen Spaltung der Ethylestergruppen wurde die Reaktion nach Abführung der Hauptmenge des sich bildenden Ethanols im Vakuum fortgeführt.

| | |
|---|---|
| Ausbeute: | 700 g |
| Mₙ: | 3500 g/mol |
| [COOH]: | 0,1 mmol/g |

### Synthesebeispiel 4: zyklisierende Depolymerisation von Poly-L-milchsäure

1000 g der in den Synthesebeispielen 1 bis 3 hergestellten Poly-L-milchsäure wurden in einer Glasapparatur mit Rührer, Außenheizung und temperierbarer Vigreuxkolonne mit ebenfalls temperierbarem Kühler in einem Vakuum von 3 bis 5 mbar in Gegenwart eines Zinn(II)-Katalysators erhitzt. In Abhängigkeit von der gewählten Depolymerisationsgeschwindigkeit wurde der Reaktionsansatz dann auf 205-220 °C erhitzt und das gebildete L,L-Dilactid über die Kolonne abgeführt, wobei die Kühlertemperatur zur Vermeidung von Kristallisation auf 90 °C gehalten wurde. Von dem gebildeten Dilactid wurden Ausbeute, Carboxylgruppengehalt, optischer Drehwert (polarimetrisch) sowie der meso-Dilactid-Gehalt bestimmt (gaschromatographisch).

| | |
|---|---|
| Ausbeute: | 900 g |
| [COOH]: | 0,13 mmol/g |
| [α]: | -261° |
| meso-Dilactid: | 5,3 % |

### Synthesebeispiel 5: zyklisierende Depolymerisation von Poly-L-milchsäure

Die in Synthesebeispiel 2 hergestellte Poly-L-milchsäure wurde im flüssig-beheizten 25-1-Laborrührreaktor durch Erhöhung der Temperatur auf 210 °C und weitere Reduzierung des Drucks auf 3 bis 5 mbar depolymerisiert und das gebildete L,L-Dilactid über die Kolonne abgeführt, wobei die Kühlertemperatur zur Vermeidung von Kristallisation auf 90 °C gehalten wird. Die Reaktion wurde bei ca. 75 % Umsatz beendet, um die Versuche problemlos ohne Entleerung und aufwändige Reinigung des Reaktors durch Wiederbefüllung mit wasserfreier L-Milchsäure mehrmals wiederholen zu können.

| | |
|---|---|
| Ausbeute: | 9,0 kg |
| [COOH]: | 0,25 mmol/g |
| [α]: | -257° |
| meso-Dilactid: | 5,0 % |

### Verqleichsbeispiel 1: Fraktionierung von Rohlactid, diskontinuierlich

500 g des in Synthesebeispiel 4 und 5 hergestellten Rohlactids wurden bei 140 bis 160 °C (Temperaturprogramm für die Aufheizphase) im Vakuum (Inertgas, N₂) bei einem Druck von 10 mbar destilliert. Die Vakuumdestillation wurde in einem Zweihalskolben mit Kapillare unter Inertgas und unter Einsatz einer temperierbaren Vigreuxkolonne durchgeführt. Nach Abtrennen des Vorlaufs (L-Milchsäure, Wasser) wurde bis zum Anstieg der Kopftemperatur auf ca. 138 °C der Hauptlauf (Dilactidgemisch) abgenommen. Der Hauptlauf wurde ein zweites Mal destilliert. Das Rücklaufverhältnis wurde bei beiden Destillationen auf 0,8-1 eingestellt.

**Tabelle 9: L,L-Dilactid nach zweimaliger Vakuumdestillation.**

| **Fraktionierung** | **Ausbeute [g]** | **[COOH] [µmol/g]** | **[α] [°]** | **meso-Dilactid [%]** |
|---|---|---|---|---|
| Ausgang | 500 | 130 | -261 | 5,3 |
| Destillation 1 | 465 | 30 | -272 | 4,8 |
| Destillation 2 | 440 | 10 | -274 | 4,8 |

### Beispiel 1: Fraktionierung von Rohlactid, diskontinuierlich

Analog zu Vergleichsbeispiel 1 wurden 500 g Rohlactid zweimal destilliert. Das über die Kapillare zugeführte Inertgas wurde in der zweiten Destillationstufe mit Wasserdampf befeuchtet, um das im Dilactidgemisch enthaltene meso-Isomer hydrolytisch zu spalten. Für die partielle Hydrolyse des gaschromatographisch bestimmten meso-Isomer-Anteils von 26,6 g wurden 3,2 g H₂O mit dem Inertgas in die Dilactidschmelze eingetragen.

**Tabelle 10: L,L-Dilactid nach zweimaliger Vakuumdestillation unter Zufuhr von befeuchtetem Inertgas.**

| **Fraktionierung** | **Ausbeute [g]** | **[COOH] [µmol/g]** | **[α] [°]** | **meso-Dilactid [%]** |
|---|---|---|---|---|
| Ausgang | 500 | 130 | -261 | 5,3 |
| Destillation 1 | 450 | 30 | -278 | 0,8 |
| Destillation 2 | 425 | 10 | -280 | 0,7 |

### Beispiel 2: zyklisierende Depolymerisation von Poly-L-milchsäure, kombiniert mit reaktiver Fraktionierung des Rohlactids, semi-kontinuierlich

Das in Synthesebeispiel 5 hergestellte Dilactidgemisch wurde in dem flüssig-beheizten 25-1-Laborrührreaktor durch Erhöhung der Temperatur auf 210°C und weitere Reduzierung des Drucks auf 3 bis 5 mbar depolymerisiert und das gebildete Dilactidgemisch über die direkt auf dem Reaktor montierte Kolonne als Kopfprodukt über eine mantelbeheizte Zuleitung (Manteltemperatur 140-160 °C) einer zweiten Kolonne (Füllkörperkolonne) zugeführt. Die Zuleitung war mit einem statischen Mischer sowie einem Ventil zur Zudosierung des für die Hydrolyse benötigten Wasserdampfs ausgerüstet. Die über Kopf abgenommenen Dilactidbrüden wurden der Füllkörperkolonne im mittleren Bereich zugeführt, um eine optimale Abtrennung der weniger (lineares Dimer) bzw. leichter flüchtigen Bestandteile (Milchsäure, überschüssiges H₂O) vom meso-isomerenfreien L,L-Dilactid zu erreichen. Als Kopfprodukt wurden der Füllkörperkolonne L-Milchsäure sowie Restwasser abgenommen. Das meso-isomerenfreie L,L-Dilactid wurde unterhalb des Brüdeneingangs ausgeschleust und kann in Abhängigkeit von der geforderten Reinheit einer nochmaligen Rektifikation unterzogen werden. Die linearen Oligomere wurden am Kolonnensumpf abgenommen und können gemeinsam mit frischer L-Milchsäure polykondensiert und anschließend erneut zyklisierend depolymerisiert werden.

In diesem System und bei den gewählten Bedingungen wurden 5 kg/h Dilactidgemisch über die erste Kolonne ausgetragen. Dies bedeutet, dass 31,25 g/h erhitztes Wasser bzw. Dampf den Brüden zudosiert werden mussten.

**Tabelle 11: L,L-Dilactid nach semikontinuierlicher reaktiver Fraktionierung**

| **Fraktionierung** | **Ausbeute [g]** | **[COOH] [µmol/g]** | **[α] [°]** | **meso-Dilactid [%]** |
|---|---|---|---|---|
| Ausgang | 9000 | 250 | -257 | 5,0 |
| Destillation 1 | 8250 | 30 | -278 | 0,8 |
| Destillation 2 | 7900 | 8 | -280 | 0,3 |

### Beispiel 3: Ringöffnungspolymerisation, diskontinuierlich in einem Glasreaktor

36 g von in Beispiel 2 hergestelltem und gereinigtem L,L-Dilactid wurden in einem zylindrischen Glasreaktor mit einem randgängigen geschraubten Blattrührer (Schraubenrührer) unter Inertgas in einem Temperierbad aufgeschmolzen. Der aus Glas gefertigte und bis zum Boden reichende Schraubenrührer durchmischte die Schmelze axial. Der Rührer wurde mit einer Drehzahl von 100 min⁻¹ betrieben. Nach Erreichen der Solltemperatur wurden zur gerührten Monomerschmelze 7,5 x 10⁻⁵ mol/mol Sn(oct)₂ als 0,1%ige Lösung in Toluol zugesetzt. Zur Ermittlung des Polymerisationsverlaufs wurden der Schmelze über einen seitlichen Ansatz Proben entnommen, von denen Momonerumsatz und Molmasse bestimmt wurden. Die Umsatzbestimmung erfolgte gravimetrisch durch Umfällen der Polymilchsäure aus Chloroform als Lösungsmittel mit einem Methanol/Diethylether-Gemisch als Fällungsmittel. Die Molmassen wurden mittels GPC in CH₂Cl₂ ermittelt. Zur Kalibrierung wurden Polystyrolstandards verwendet.

Nach einer Polymerisationszeit von 20 min wurden ermittelt:

| | |
|---|---|
| Monomerumsatz : | 96 % |
| Molmasse: | Mₙ = 80.000 g/mol |
| | M_{w} = 165.000 g/mol |
| Polydispersität: | M_{w}/Mₙ = 2,08 |

### Beispiel 4: Ringöffnungspolymerisation, kontinuierlich in einer zweistufigen apparativen Anordnung aus Rührreaktor und Doppelschneckenextruder

Für die Massepolymerisation des L,L-Dilactids durch einen Reaktivextrusionsprozess wurde ein dichtkämmender, gleichsinnig drehender Doppelschneckenextruder mit Innentemperatur- und Drehmomentregelung verwendet. Der eingesetzte Doppelschneckenextruder wies ein (UD)-Verhältnis von 35 mit modular aufgebauter Schnecke auf, um flexibel die Schneckenkonfigurierung hinsichtlich Transport-, Knet- und Stauelementen den Prozessbedingungen und Endproduktparametern anpassen zu können. 1000 g von in Beispiel 2 erhaltenem L,L-Dilactid wurden mit einer Lösung von Zinn(II)-octanoat in Toluol in einem Labortaumelmischer intensiv vermischt, und anschließend wurde das Toluol im Vakuum abgezogen. Die Katalysatorkonzentration betrug 2,0 x 10⁻⁴ mol/mol und entsprach einem Zinngehalt von 165 ppm. Die Zudosierung des katalysatorhaltigen Dilactids erfolgte mittels volumetrischer Dosierung über Dosierschnecken.

| | |
|---|---|
| Monomerumsatz : | 94 % |
| Molmasse: | Mₙ = 112.000 g/mol |
| | M_{w} = 239.000 g/mol |
| Polydispersität: | M_{w}/Mₙ = 2,13 |

Die obigen Beispiele belegen klar, dass durch das erfindungsgemäße Reinigungsverfahren stereoisomerenreine Dilactide erhältlich sind, die ohne weitere Reinigung in Ringöffnungspolymerisationsreaktionen zum Erhalt von äußerst hochmolekularer Polymilchsäure eingesetzt werden können.

## Patentansprüche

1. Verfahren zur Reinigung von L,L- oder D,D-Dilactid aus einem Stereoisomerengemisch, wobei ein die Dilactid-Stereoisomere sowie höher und/oder niedriger siedende Bestandteile umfassendes Gemisch einem ersten Destillationsschritt unterzogen wird, um eine von den höher und niedriger siedenden Bestandteilen des Gemischs weitgehend gereinigte Fraktion der Dilactid-Stereoisomere zu erhalten, die anschließend einem zweiten Destillationsschritt unterzogen wird, um im Wesentlichen stereoisomerenfreies, gereinigtes L,L- oder D,D-Dilactid zu erhalten,
**dadurch gekennzeichnet, dass** vor dem zweiten Destillationsschritt eine aus dem Gehalt an meso-Dilactid im Isomerengemisch berechnete, stöchiometrische Menge oder ein geringer Überschuss an Wasser oder Wasserdampf zu der Fraktion der Dilactid-Stereoisomere zugesetzt wird, um meso-Dilactid selektiv zu Lactoylmilchsäure und/oder zu L- und D-Milchsäure zu hydrolysieren, von denen das L,L- oder D,D-Dilactid im zweiten Destillationsschritt in im Wesentlichen reiner Form abgetrennt wird.

2. Verfahren nach Anspruch 1, **dadurch gekennzeichnet, dass** das dem ersten Destillationsschritt unterzogene Gemisch aus einem Depolymerisationsreaktor zugeführt wird.

3. Verfahren nach Anspruche 1 oder 2, **dadurch gekennzeichnet, dass** nach dem zweiten Destillationsschritt ein zusätzlicher, dritter Destillationsschritt zur weiteren Reinigung des L,L- oder D,D-Dilactids durchgeführt wird.

4. Verfahren nach einem der Ansprüche 1 bis 3, **dadurch gekennzeichnet, dass** das Wasser oder der Wasserdampf in eine Verbindungsleitung zwischen erster und zweiter Destillationsstufe zugesetzt wird.

5. Verfahren nach Anspruch 4, **dadurch gekennzeichnet, dass** die Dilactid-Stereoisomer-Fraktion und das Wasser oder der Wasserdampf in der Verbindungsleitung mit einer Mischeinrichtung durchmischt werden.

6. Verfahren nach einem der vorangegangenen Ansprüche, **dadurch gekennzeichnet, dass** zumindest ein Destillationsschritt in einer Rektifikationskolonne durchgeführt wird.

7. Verfahren nach einem der vorangegangenen Ansprüche, **dadurch gekennzeichnet, dass** zumindest ein Destillationsschritt bei einer Temperatur von 100 bis 200 °C, vorzugsweise 120 bis 180 °C, insbesondere 140 bis 160 °C, durchgeführt wird.

8. Verfahren nach einem der vorangegangenen Ansprüche, **dadurch gekennzeichnet, dass** zumindest ein Destillationsschritt bei einem Druck von 1 bis 100 mbar, vorzugsweise 1 bis 50 mbar, insbesondere 3 bis 30 mbar, durchgeführt wird.

9. Verfahren nach einem der vorangegangenen Ansprüche, **dadurch gekennzeichnet, dass** zumindest ein Destillationsschritt unter Inertgas durchgeführt wird.

## Claims

1. A process for purifying L- or D-lactide from a mixture of stereoisomers, wherein a mixture comprising the lactide stereoisomers as well as higher and/or lower boiling components is subjected to a first distillation step in order to obtain a fraction containing said lactide stereoisomers and being largely free of the higher and lower boiling components of the mixture, said fraction being subsequently subjected to a second distillation step in order to obtain purified L- or D-lactide which is essentially free of stereoisomers,
**characterized in that** a stoichiometric amount or a slight excess of water or water vapor, which is calculated from the amount of meso-lactide in the isomeric mixture, is added to said fraction containing the lactide stereoisomers before the second distillation step in order to selectively hydrolyze meso-lactide into lactoyl lactic acid and/or into L- and D-lactic acid, from which the L- or D-lactide is separated in the second distillation step in an essentially pure form.

2. The process according to claim 1, **characterized in that** the mixture to be subjected to said first distillation step is supplied from a de-polymerization reactor.

3. The process according to claim 1 or claim 2, **characterized in that** an additional third distillation step is carried out after the second distillation step in order to purify the L- or D-lactide.

4. The process according to any one of the claims 1 to 3, **characterized in that** the water or the water vapor is added into a conduit connecting the first and second distillation steps.

5. The process according to claim 4, **characterized in that** the lactide stereoisomeric fraction and the water or the water vapor are mixed in the connecting conduit by means of a mixing device.

6. The process according to any one of the preceding claims, **characterized in that** at least one distillation step is carried out in a rectification column.

7. The process according to any one of the preceding claims, **characterized in that** at least one distillation step is carried out at a temperature of 100 to 200 °C, preferably 120 to 180 °C, especially 140 to 160 °C.

8. The process according to any one of the preceding claims, **characterized in that** at least one distillation step is carried out at a pressure of 1 to 100 mbar, preferably 1 to 50 mbar, especially 3 to 30 mbar.

9. The process according to any one of the preceding claims, **characterized in that** at least one distillation step is carried out in an inert gas.

## Revendications

1. Procédé pour la purification de L- ou D-lactide d'un mélange de stéréoisomères, un mélange comprenant lesdits stéréoisomères du lactide ainsi que des composants à point d'ébullition plus élevé ou plus bas étant soumis à une première étape de distillation pour obtenir une fraction des stéréoisomères du lactide qui est largement purifiée desdits composants à point d'ébullition plus élevé ou plus bas et qui est ensuite soumise à une deuxième étape de distillation pour obtenir un L- ou D-lactide sensiblement sans stéréoisomères,
**caractérisé en ce qu'**une quantité stoechiométrique ou un faible excès d'eau ou de vapeur d'eau, calculée sur la base de la teneur en méso-lactide dudit mélange d'isomères, est ajouté(e) à ladite fraction des stéréoisomères du lactide pour hydrolyser le méso-lactide d'une manière sélective pour obtenir de l'acide lactoyllactique et/ou de l'acide L-lactique et de l'acide D-lactique dont du L- ou du D-lactide est séparé dans une forme essentiellement pure dans ladite deuxième étape de distillation.

2. Procédé selon la revendication 1, **caractérisé en ce que** ledit mélange qui est soumis à la première étape de distillation est amené d'un réacteur de dépolymérisation.

3. Procédé selon la revendication 1 ou 2, **caractérisé en ce que** une troisième étape de distillation supplémentaire est exécutée après la deuxième étape de distillation pour une purification supplémentaire du L- ou D-lactide.

4. Procédé selon l'une des revendications 1 à 3, **caractérisé en ce que** l'eau ou le vapeur d'eau est ajouté dans une conduite de connexion entre la première et la deuxième étape de distillation.

5. Procédé selon la revendication 4, **caractérisé en ce que** la fraction des stéréoisomères du lactide et l'eau ou le vapeur d'eau sont mélangés dans la conduite de connexion par un mélangeur.

6. Procédé selon l'une des revendications précédentes, **caractérisé en ce qu'**au moins une étape de distillation est exécutée dans une colonne de rectification.

7. Procédé selon l'une des revendications précédentes, **caractérisé en ce qu'**au moins une étape est exécutée à une température de 100 à 200 °C, de préférence de 120 à 180 °C, notamment de 140 à 160 °C.

8. Procédé selon l'une des revendications précédentes, **caractérisé en ce qu'**au moins une étape est exécutée sous une pression de 1 à 100 mbar, de préférence de 1 à 50 mbar, notamment de 3 à 30 mbar.

9. Procédé selon l'une des revendications précédentes, **caractérisé en ce qu'**au moins une étape est exécutée sous un gaz inerte.
